# EUROPEAN PATENT APPLICATION

(11) **EP 0 037 133 A1**
(43) Date of publication of application: **07.10.1981**
(21) Application number: 81200262.4
(22) Date of filing: 09.03.1981
(51) Int. Cl.: A01N 43/58, C07D 237/24

(54) **Pyridazinone compounds, process for their preparation, compositions containing them and a method of regulating the growth of plants, increasing the yield of soya bean plants and sterilizing the male anthers of plants, including small grain cereal plants, using them as well as a method of producing F1 hybrid seed**

(30) Priority: 26.03.1980 GB 8010193
(71) Applicant: SHELL INTERNATIONALE RESEARCH MAATSCHAPPIJ B.V., NL-2596 HR Den Haag (NL)
(72) Inventor: Haddock, Ernest, Sheerness Sheppey Kent (GB); Hopwood, William John, Sittingbourne Kent (GB)
(74) Representative: Hunter, Keith Roger Ian

(57) **Abstract**

A compound of the general formula 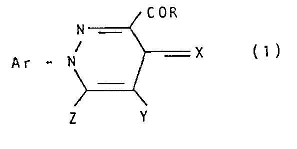 in which X represents an oxygen or sulphur atom;
Y represents a hydrogen or halogen atom or an alkyl group; Z represents an alkyl group;
Ar represents an optionally-substituted phenyl group; and R represents a group
   i) NR¹R² or ONR¹R², in which R' represents a hydrogen atom or an alkyl, alkenyl, alkynyl or cycloalkyl group and R' represents a hydrogen atom, an alkyl, alkenyl, alkynyl, cycloalkyl, alkoxy or hydroxy group, or an acyl group derived from a carboxylic or carbamic acid, or an alkyl group substituted with a carboxylic acid or ester group, or R' and R² together with the interjacent nitrogen atom represent an optionally substituted heterocyclic ring; or
   ii) OR³ in which R³ represents an alkenyl, alkynyl, cycloalkyl or aryl group or an alkyl, alkenyl, alkynyl, cycloalkyl or aryl group substituted by one or more of the same or different substituents selected from halogen atoms, alkoxy, alkylthio, cyano and aryl groups, acyl groups derived from carboxlic or carbamic acids, groups of formula NR¹R² where R¹ and R² have the meaning given above, and carboxy groups and salts and esters thereof; or
   iii) SR⁴ in which R⁴ has one of the meanings given above for R³ or represents a hydrogen atom or an alkyl group; or iv) ON=CR⁴R⁵ in which R⁴ has the meaning given above and R⁵ has one of the meanings given above for R³ or represents an alkyl group, or R⁴ and R⁵ together with the interjacent carbon atom represent an optionally substituted carbocyclic or heterocyclic ring; or
   v) NR⁶NR⁷R⁸ in which each of R⁶ and R⁷ independently has one of the meanings given above for R¹ and R⁸ has one of the meanings given above for R' or represents an acyl group derived from a carboxylic acid or R⁷ and R⁸ together with the interjacent nitrogen atom represent an optionally substituted heterocyclic ring; have useful plant growth regulating properties.

## Description

This invention relates to pyridazinone compounds, process for their preparation, compositions containing them and a method of regulating the growth of plants using them.

In the past, the vast majority of agricultural chemical research work was directed towards the development of chemical fertilizers and the development of chemicals to combat pests, diseases and weeds which reduce the yield of crops. The belief was that by adequately feeding the plant and by optimizing its environment, the yields of the crop could be maximized.

In recent years it has become clear that it is possible to affect the growth of plants in a much more fundamental way, that is by the application of chemicals which affect the biochemical processes occuring in the plant. Much research is now being directed towards the development of chemical plant growth regu- lants which are capable of altering the biochemistry of plants in such a way that useful effects are achieved. One important field of research is into the stimulation of crops to produce higher yields. It would for example be most useful to be able to increase the yield of soyabeans, a valuable source of protein, by means of a chemical treatment.

The invention provides a compound of the general formula 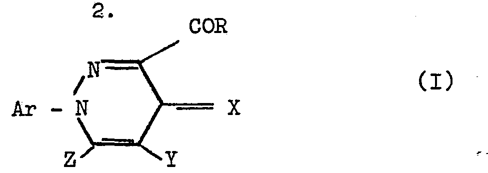
in which X represents an oxygen or sulphur atom;
Y represents an hydrogen or halogen atom or an alkyl group;
Z represents an alkyl group;
Ar represents an optionally-substituted phenyl group; and R represents a group
   i) NR¹R² or ONR¹R², in which R represents a hydrogen atom or an alkyl, alkenyl, alkynyl or cycloalkyl group and R² represents a hydrogen atom, an alkyl, alkenyl, alkynyl, cycloalkyl, alkoxy or hydroxy group, or an acyl group derived from a carboxylic or carbamic acid, or an alkyl group substituted with a carboxylic acid or ester group, or R¹ and R² together with the interjacent nitrogen atom represent an optionally substituted heterocyclic ring; or
   ii) OR³ in which R³ represents an alkenyl, alkynyl, cycloalkyl or aryl group or an alkyl, alkenyl, alkynyl, cycloalkyl or aryl group substituted by one or more of the same or different substituents selected from halogen atoms, alkoxy, alkylthio, cyano and aryl groups, acyl groups derived from carboxylic or carbamic acids, groups of formula NR¹R² where R1 and R² have the meaning given above, and carboxy groups and salts and esters thereof; or
   iii) SR⁴ in which R⁴ has one of the meanings given above for R³ or represents a hydrogen atom or an alkyl group; or iv) ON=CR⁴R⁵ in which R4 has the meaning given above and R5 has one of the meanings given above for R³ or represents an alkyl group, or R⁴ and R⁵ together with the interjacent carbon atom represent an optionally substituted carbocyclic or heterocyclic ring; or
   ᵥ) NR⁶NR⁷R⁸ in which each of R⁶ and R⁷ independently has one of the meanings given above for R and R has one of the meanings given above for R or represents an acyl group derived from a carboxylic acid, or R⁷ and R⁸ together with the interjacent nitrogen atom represent an optionally substituted heterocyclic ring.

Unless otherwise stated, any alkoxy, alkylthio, alkyl, alkenyl or alkynyl group in a compound of the general formula I preferably has up to 6, especially up to 4, carbon atoms. A cycloalkyl group preferably has 3, 5 or 6 carbon atoms. An aryl group is preferably a phenyl group. An acyl group is preferably one of the general formula COR⁹ or CONHR⁹ in which R⁹ is an alkyl or cycloalkyl group or an optionally substituted phenyl group in which the optionally subtituents are preferably halogen atoms, alkyl groups and/or alkoxy groups.

A carbocyclic ring preferably has 5 or 6 carbon atoms. A heterocyclic ring preferably has 5 or 6 ring atoms of which 1, 2 or 3 are oxygen, sulphur and/or nitrogen, nitrogen atoms being prefered. Carbocyclic and heterocyclic rings may be saturated or unsaturated, and are preferably unsubstituted or substituted by one or more substituents selected from halogen atoms, alkyl groups and alkoxy groups.

Preferably X represents an oxygen atom. Preferably Y represents a hydrogen atom. Preferably Z represents a methyl group. Preferably Ar represents a phenyl group which is unsubstituted or substituted by one or more of the same of different subtituents selected from halogen atoms and alkyl, alkoxy, alkylthio, cyano, aryl and carboxy groups. More preferably, Ar represents a phenyl group which is unsubstituted or substituted by one or more halogen atoms and/or methyl groups. An especially prefered group Ar is a monohalophenyl group, especially a 4-chlorophenyl group.

In a group of formula NR¹R² or ONR¹R², R¹ preferably represents a hydrogen atom, an alkyl, especially methyl, group, or a cycloalkyl, especially cyclohexyl, group and R 2 represents a hydrogen atom, an alkyl, especially methyl, group, an alkoxy, especially methoxy, group, a hydroxy group, or a group of formula COR⁹ or C_{O}.N_{HR}⁹ where R⁹ is an alkyl, especially methyl, or cycloalkyl, especially cyclohexyl, group; or -CH-COOR¹¹ where R¹⁰ is a hydrogen atom or an alkyl group, especially methyl, and R¹¹ is a hydrogen atom or an alkyl group of up to 3 carbon atoms, especially ethyl; or R¹ and R² together with the interjacent nitrogen atom represent a 5 or 6 membered heterocyclic ring having 2 or 3 nitrogen atoms, for example an imidazole or triazole ring.

In a group of formula OR³, R preferably represents an alkenyl group preferably having up to 4 carbon atoms, a cycloalkyl group preferably having 3, 5 or 6 carbon atoms, or an alkyl or alkenyl group, preferably having up to 4 carbon atoms, substituted by one or more, especially 1 or 2, halogen atoms, by a group of formula COR⁹ where R⁹ has the meaning given above, by a phenyl group, by an alkoxy group, by an NR¹R² group where R and R² suitably have the preferred meanings given above, or by a carboxy group or a salt or ester thereof. Preferred salts are alkali metal salts or optionally alkyl-substituted ammonium salts, and prefered esters are alkyl esters.

More preferably R ³ represents an alkenyl group, for example an allyl group, an cycloalkyl group, for example a cyclohexyl group, a monohaloalkyl group, a benzyl group, or a group -CH(CH₃)-or-(CH₂)ₙ- where n is 1 to 4, bearing an alkylcarbonyl group, an alkoxy group, an NR¹R² group or a carboxy group or a salt or ester thereof.

In a group of formula SR⁴, R⁴ suitably has one of the preferred meanings given above for R³, or represents a hydrogen atom or an alkyl group preferably having up to 4 carbon atoms, for example a methyl, ethyl or isopropyl group.

In a group of formula ON=CR⁴R⁵, R⁴ suitably has one of the preferred meanings given above, and R suitably has one of the preferred meanings given above for R³ or represents an alkyl, especially methyl, group.

In a group of formula NR⁶NR⁷R⁸, each of R , R and R preferably independently has one of the preferred meanings given for R¹ above or R⁷ and R⁸ together with the interjacent nitrogen atom represent a heterocyclic ring having 1, 2 or 3 nitrogen atoms, for example an imidazole or triazole ring.

Especially preferred compounds of the general formula I are those in which R represents a group of general formula
i) NR¹R² in which R¹ represents a hydrogen atom or a methyl or cyclohexyl group, and R ² represents a methoxy group or a -CO.NH cyclohexyl group; or R ¹ and R² together with the interjacent nitrogen atom represent an imidazole ring; or
ii) OR³ in which R³ represents an allyl, 2-chloroethyl, 2-bromoethyl or methylcarbonylmethyl group; or
iii) SR4 in which R⁴ represents an isopropyl or cyclohexyl group; or
iv) ON=CR⁴R⁵ in which each of R⁴ and R represents a methyl group; or
ᵥ) _{NR}⁶_{NR}⁷_{R}⁸ in which R⁶ represents a hydrogen atom and each of R⁷ and R⁸ represents a hydrogen atom or a methyl group.

The invention also provides a process for the preparation of a compound of the general formula I, in one or more steps, which involves converting a compound of the general formula 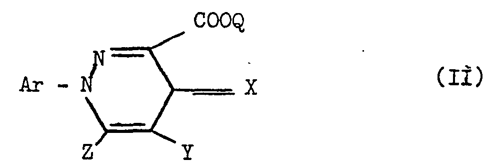 in which Ar, X, Y and Z have the meanings given for the general formula I and Q represents a hydrogen atom, a cation or an alkyl group, into a compound of the general formula I by reaction with a compound capable of replacing the group OQ with a group R where R has the meaning given for the general formula I.

It may in some cases be desirable to prepare one compound of the general formula I and then convert said compound into another compound of formula I.

The process according to the invention is, in general terms, an esterification, transesterification or amidation reaction, and may be carried out under the conditions usual for such reactions. It is often useful to start from the free acid of formula II and to convert it into a reactive functional derivative thereof. An acid halide, especially the acid chloride, is often a useful reactive derivative. A mixed anhydride may also be very useful. Generally, mixed anhydrides may be prepared by reacting the free acid or, preferably, an amine salt, for example the triethylamine salt thereof, with an ester of chloroformic acid, to give a mixed anhydride containing the grouping 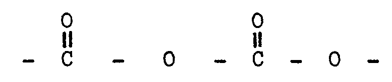 Common chloroformate esters are the benzyl ester and alkyl esters, for example the ethyl ester or isobutyl ester. In some cases, a reactive functional derivative which is itself a compound of the general formula I according to the invention may be prepared and subsequently converted into another compound of the general formula I. Compounds of the general formula I in which R represents an imidazole ring bonded through a nitrogen atom, are especially useful in this respect.

The process of the invention is suitably carried out in the presence of a suitable inert solvent, for example a hydrocarbon or a halogented hydrocarbon. Water may be suitable in some cases. The reaction temperature may vary widely depending on the reactants used, but it is suitably in the range from 0 to 150 C. It may in some cases be most convenient to conduct the reaction at room temperature or at the reflux temperature of the solvent used.

Suitable compounds capable of replacing the OQ group by a group R in the process according to the invention, include:-
i) compounds of formula NHR¹R² or HONR-R2.
ⁱi) compounds of formula R³OH or R³Hal where Hal is a halogen atom;
iii) compounds of formula R4SH;
iv) compounds of formula HON=_{CR}⁵_{R}⁶ or alkali metal salts thereof; and
V) compounds of formula _{NHR}⁷_{NR}⁸_{R}⁹.

Thus compounds of general formula I in which R is an NR¹R² or ONR¹R² group, may be prepared by reacting an acid of formula II or a functional derivative thereof with an amine or hydroxylamine of formula NHR¹R² or HO.NR¹R². The acid chloride is a useful functional derivative. Alternatively, the acid may be reacted directly with carbonyl diimidazole to produce the compound of general formula I in which R represents an N-bonded imidazole group, and if desired, said compound may be further reacted with an appropriate amine or hydroxylamine to produce a second compound of general formula I. An especially preferred method of preparing compounds in which R represents ONR¹R² is by the mixed anhydride technique described above, followed by reaction with the appropriate hydroxylamine.

Compounds of general formula I in which R is an OR³ group may be prepared by any suitable esterification or transesterification process.

For example, an alcohol of formula R³OH may be reacted directly with a free acid of formula II or with a reactive derivative thereof, such as the acid chloride or the imidazole derivative described above. Especially for substituted alkyl esters, it is preferred to react a compound of formula II in which Q is a cation, especially an alkali metal ion, with a compound of formula R Hal where Hal is a halogen, especially chlorine, atom.

Compounds in which R represents SR4 may be prepared by the same general methods as described above using a thiol R⁴SH instead of an alcohol R³0H, or, alternatively, by transesterification. Thus an alkyl ester of formula II may be reacted with a compound of formula R⁴SH to produce the desired compound.

Compounds in which R represents ON=CR⁵R⁶ may be prepared by reacting a functional derivative of the acid of formula II, suitably the acid halide or an imidazole derivative, with an ₒₓiₘe of formula HON=CR⁵R⁶ or, preferably, an alkali metal salt thereof. Alternatively, the mixed anhydride technique described above may be used.

Compounds in which R represents NR⁷NR⁸R⁹ are most suitably prepared by reacting an alkyl ester of formula II or functional derivative of an acid of formual II, with a hydrazide of general formula _{HNR}⁷_{NR}⁸_{R}⁹_{.}

The compounds according to the invention exhibit useful plant growth regulating effects, and the invention therefore provides a plant growth regulating composition which comprises a compound of the general formula I together with a suitable carrier. The invention also provides a method of regulating the growth of a plant, which comprises treating the plant, the seed from which it is to be grown, or the medium in which it is growing or to be grown, with a compound or a composition according to the invention.

Different plant growth regulating effects will be obtained depending on the growth stage of the plant when treated. One preferred embodiment of the invention is a method of increasing the yield of soyabeans, which comprises treating a soyabean plant or the soil in which it is growing with a compound or a composition according to the invention, the treatment being carried out at a growth stage after the flowers of the plant have differentiated. Generally the treatment may be carried out from the time at which the flowers of the plant have differentiated, and throughout the time when the flowers are open.

A second preferred embodiment of the invention is a method of sterilizing the male anthers of a plant, which comprises treating the plant or the soil in which it is growing with a compound or a composition according to the invention, the treatment being carried out at a growth stage such that sterilization occurs.

This second embodient of the invention makes it possible to sterilize the male anthers of plants, especially small-grain cereal plants, without substantially affecting female fertility. The resulting male-sterile plant may then be cross-pollinated by pollen from a plant of a different strain, producing F₁ hybrid seed. This process is especially useful for self-pollinating plants. Thus the invention also provides a method of producing F₁ hybrid seed which comprises cross-pollinating a plant which has been treated by the sterilizing process of the present invention with a second plant of a different strain.

The appropriate growth stage for carrying out the sterilizing process will vary from species to species, but generally it is appropriate to treat the plant at a growth stage before or during the earlier stages of flower differentation. For small-grain cereal plants, such as wheat or barley, the treatment is most suitably carried out when the plant is at a stage of growth between late tillering and emergence of the ear.

Preferably the growth of plants is regulated by the application of the active compound at a dosage in the range of from 0.05 to 2 kg/ha, preferably 0.25 to 1 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used.

Suitable solid carriers include natural and synthetic clays and silicates, for example-natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones,-for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution.

A surface-active agent may be an emulsifying--agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3-10% w of a dispersing agent and, where necessary, 0-10% w of stabiliser(s) and/or other additives such as penetrants or stickers. Dusts are usually formulated as a dust concentrate having a similar compositions to that of a wettable powder but without a dispersant, and are diluted in the field with further solid carrier to give a composition usually containing g-10% w of active ingredient. Granules are usually prepared to have a size between 10 and 100 BS mesh (1.676 - 0.152 mm), and may be manufactured by agglomeration or impregnation techniques. Generally, granules will contain z-25% w active ingredient and 0-10% w of additives such as stabilisers, slow release modifiers and binding agents. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'- like consistency.

The compositions of the invention may also contain other ingredients, for example, other compounds possessing plant growth regulating, insecticidal or fungicidal-properties. The following Examples illustrate the invention.

### Example 1 Preparation of the 2-propanone oxime ester of 1-(4-chlorophenyl)-3-carboxy-6-methylpyridazin-4-one

1-(4-chlorophenyl)-3-carboxy-6-methylpyridazin-4-one (2.6 g) was dissolved in methylene chloride (125 ml) and carbonyldiimidazole (2 g) was added. The solution was stirred at room temperature for 2 hours, and 2-propanone oxime (0.7 g) was then added. Stirring was continued for a further 30 hours. The solution was then evaporated down, and the residue was treated with water and extracted with ethyl acetate. The organic extract was washed with sodium bicarbonate solution and then with water, dried over sodium sulphate, and evaporated to dryness. The resulting white solid was recrystallized from a 1:1 mixture of ethyl acetate and petrol (boiling point 60-80°C) to give 2.1 g of the desired product, melting point 105-107 C.

### Elemental Analysis

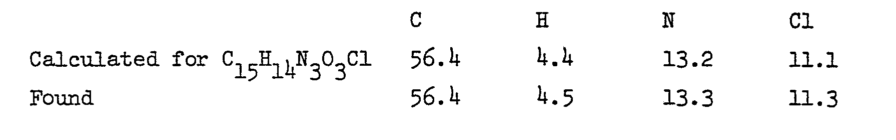

### Examples 2 and 3

The procedure of Example 1 was repeated except that the 2-propanone oxime was replaced by isopropylthiol and by cyclohexyl- thiol.

### Example 2 1-(4-chlorophenyl)-3-isopropylthiocarbonyl-6-methylpyridazin-4-one

Melting point 165-167°C.

### Elemental Analysis

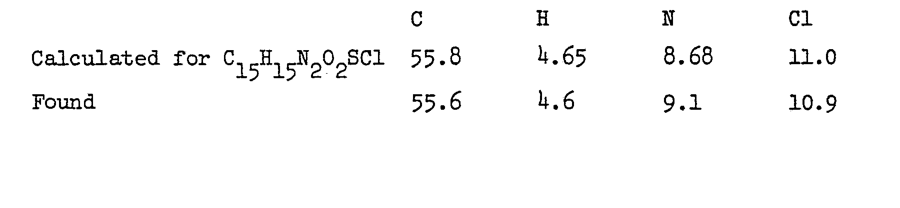

Example 3 1-(4-chlorophenyl)-3-cyclohexylthiocarbonyl-6-methylpyridazin-4-one

Melting point 188-190°C.

### Elemental Analysis

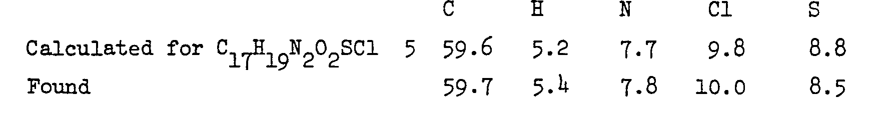

### Example 4 Preparation of 1-(4-chlorophenyl)-3-(2-propenylox- ycarbonyl)-6-methylpyridazin-4-one

1-(4-chlorophenyl)-3-carboxy-6-methylpyridazin-4-one (5 g) and para-toluene sulphonic acid (0.25 g) were added to allyl alcohol (20 ml) and heated for 1½ hours under reflux. 200 ml of benzene were then added and the mixture was refluxed for a further 3 hours. It was then stripped of solvent on a rotary evaporator and the residue was taken up in methylene chloride. The solution was washed with saturated sodium bicarbonate solution and then with water, dried over sodium sulphate, and evaporated down. After recrystallization from toluene, 2.2 g of the desired product, melting point 105-106°C, were obtained.

### Elemental Analysis

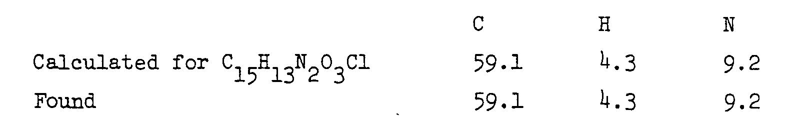

### Example 5 Preparation of the hydrazide of 1-(4-chlorophenyl)-3-carboxy-6-methylpyridazin-4-one

1-(4-chlorophenyl)-3-methoxycarbonyl-6-methypyridazin-4-one (1 g) was dissolved in a 1:1 mixture of methanol and water (20 ml) and hydrazine (1 ml) was added. The mixture was stirred for 3 hours, during which period a white solid separated. This solid was filtered off and recrystallized from a 1:1 mixture of methan- cl and water to give 0.95 g, corresponding to a 92% yield, of the hemihydrate of the desired product, melting at 240-242°C.

### Elemental analysis

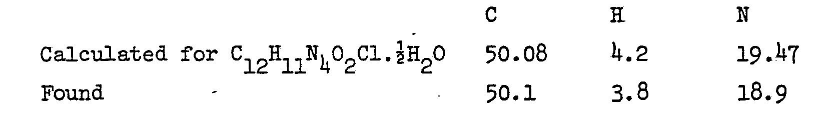

### Example 6 The N,N-dimethyloydazide of 1-(4-chlorqphenyl)-3-carboxy-6-methylpyridazin-4-one

The procedure of Example 5 was repeated using N,N-dimethylhydrazine instead of hydrazine.

Melting point of hemihydrate: 174-176°C.

### Elemental Analysis

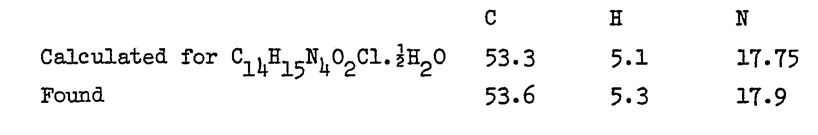

### Example 7 Preparation of 1-(4-chlorophenyl)-3-(imidazol-1- ylcarbonyl)-6-methylpyridazin-4-one

1-(4-chlorophenyl)-3-carboxy-6-methylpyridazin-4-one (5.3 g) was dissolved in methylene chloride (100 ml), and carbonyldiimidazole (4 g) was added. The mixture was stirred for 1 hour, during which time some solid separated out.

20 ml of the mixture was removed and evaporated to give 1.1 g of the desired product, melting point 82-84°C. The product was very hydroscopic, and was probably hydrated.

### Elemental Analysis

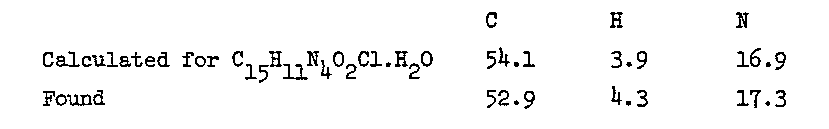

### Example 8 Preparation of 1-(4-chlorophenyl)-3-(N-methyl-N-methoxyaminocarbonyl)-6-methylpyridazin-4-one

The reaction mixture remaining after 20 ml had been removed as described in Example 7, was treated with 0,N-dimethylhydroxylamine hydrochloride (1.7 g) and trimethylamine (2.8 ml) and the mixture was stirred overnight. It was then washed successively with 2N hydrochloric acid, water, saturated sodium bicarbonate solution and water, dried over sodium sulphate and evaporated to dryness.

2.4 g of the desired compound were obtained, melting point 160-162°C.

### Elemental Analysis

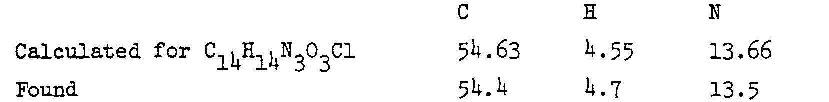

### Example 9 Preparation of 1-(4-chlorophenyl)-3-(N-methoxyamino- carbonyl)-6-methylpyridazin-4-one

The procedure of Example 8 was repeated using 0-methylhydroxylamine. The desired compound had a melting point of 168-170°C.

### Elemental Analysis

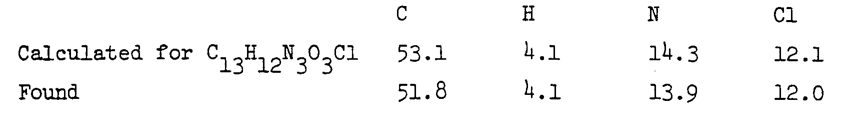

### Example 10 Preparation of 1-(4-chlorophenyl)-3-(N,N'-dicyclo hexyl aminocarbonylaminocarbonyl)-6-methylpyrid- azin-4-one

Dicyclohexylcarbodiimide (2.1 g) was added to a stirred solution of 1-(4-chlorophenyl)-3-carboxy-6-methylpyridazin-4-one (2.6 g) in methylene chloride (125 ml) and acetonitrile (125 ml). The reaction mixture was stirred at room temperature for 24 hours. A solid separated out and was filtered off. The filtrate was washed with water and then with saturated sodium bicarbonate solution, dried over sodium sulphate, filtered and evaporated to dryness. The desired product was obtained in 81% yield, and had a melting point of 142-144°C.

### Elemental Analysis

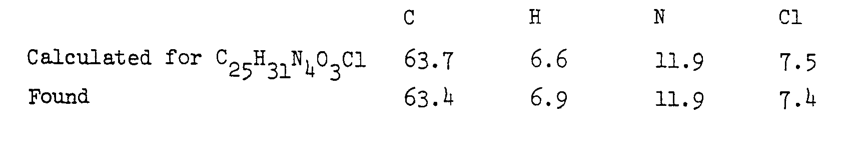

### Example 11 Preparation of 1-(4-chlorophenyl)-3-methylcarbonyl- methoxycarbonyl-6-methylpyridazin-⁴⁻one

1-(4-chlorophenyl)-3-carboxy-6-methylpyridazin-4-one (2.645g), sodium hydroxide (0.4 g), water (5 ml) and ethanol (100 ml) were refluxed together for 10 minutes. The mixture was then evaporated down and the residue was azeotroped with toluene. 50 ml of chloroacetone were added and the mixture was refluxed for 16 hours, then cooled and extracted with chloroform (500 ml). The chloroform extract was washed with water (500 ml), dried over magnesium sulphate and evaporated to give 3.0 g of a brown oil. This oil was purified by chromatography and recrystallization from ethyl acetate to give 1.0 g of the desired product as white crystals, melting point 183-185⁰C.

### Elemental Analysis

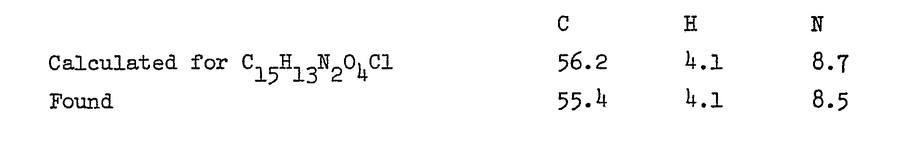

### Examples 12 and 13

The procedure of Example 11 was repeated using 1,2-dibromoethane and 1,2-dichloroethane instead of chloroacetone.

### Example 12 1-(4-chlorophenyl)-3-(2-bromoethoxycarbonyl)-6-methylpyri dazin-4-one

Melting point 181-182°C.

### Elemental Analysis

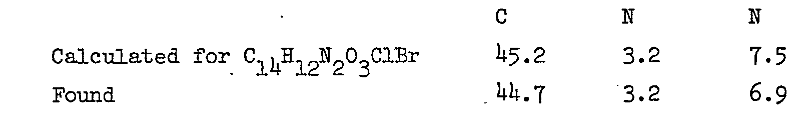

### Example 13 1-(4-chlorophenyl)-3-(2-chloroethoxycarbonyl)-6-methylpyridazin-4-one

Melting point 185-187°C.

### Elemental Analysis

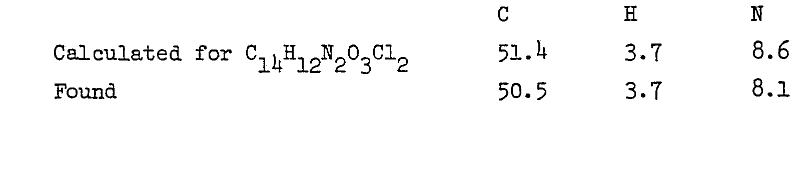

### Example 14 Alternative Preparation of the Compound of Example 2

1-(4-chlorophenyl)-3-carboxy-6-methylpyridazin-4-one (100 g) was dissolved in dry methylene chloride (1 litre), triethylamine (53 ml) was added and the solution was stirred for 15 minutes. Freshly distilled ethyl chloroformate (36.4 ml) was added dropwise over 20 minutes, the mixture being maintained between -5 and +5⁰C, and the reaction mixture was stirred for 30 minutes. Isopropylthiol (35.6 ml) was then added dropwise over 10 minutes and the reaction mixture was stirred for 12 hours.

The methylene chloride solution was washed successively with water, 2N.HC1, water and sodium bicarbonate solution, dried over sodium sulphate, filtered and evaporated. The residue was treated with diethyl ether (2 litres), and the solution was filtered and evaporated. After recrystallization from a mixture of ethyl acetate and petroleum (1.75:1) 57 g of the desired product were obtained.

### Example 15 1-(4-chlorophenyl)-3-(N-ethoxycarbonylmethylamino- carbonyl)-6-methylpyridazin-4-one

1-(4-chlorophenyl)-3-carboxy-6-methylpyridazin-4-one (7.9g) was dissolved in methylene chloride (80ml) and carbonyldiimidazole (6g) was added. This solution was stirred for 30 minutes at room temperature and a methylene chloride (40ml) solution of ethyl glycinate. HC1 (41g) and triethylamine (4.2ml) was added. This mixture was stirred for a further 20 hours after which it was washed successively with 2N hydrochloric acid, water, saturated sodium bicarbonate solution and water. The washed mixture was then dried over sodium sulphate and evaporated to dryness to afford 5.2g of the desired compound melting at 125-127°C.

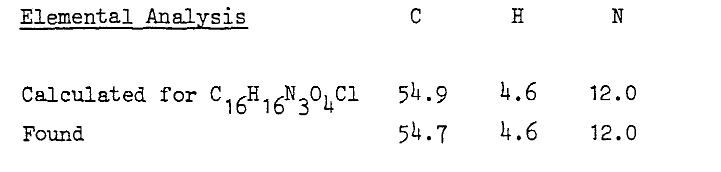

### Example 16 1-(4-chlorophenyl)-3-(N-carboxymethylaminocarbonyl)-6-methylpyridazin-4-one

A portion (2.5g) of the product of Example 15 was dissolved in ethanol (30ml), a 2N sodium hydroxide solution (30ml) was added and the resulting solution was heated on a steam bath for 15 minutes. After cooling to room temperature, the alcohol was evaporated from the solution and the resulting aqueous solution was acidified with concentrated hydrochloric acid. A buff coloured solid was obtained which on recrystallization from ethyl acetate afforded 1.6g of the desired product melting at 294--296°C.

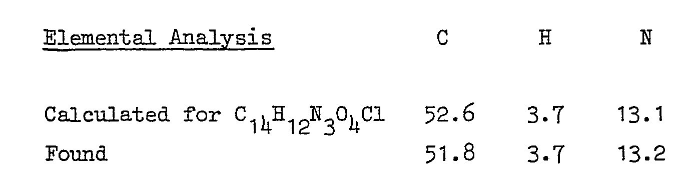

### Example 17 1-(4-chlorophenyl)-3-(N-(1-ethox,ycarbonyl)ethylaminocarbonyl]-6-methylpyridazin-4-one, hemihydrate

The procedure of Example 15 was repeated using DL-alanine ethyl ester hydrochloride instead of ethyl glycinate hydrochloride.

Melting point of hemihydrate: 134-136°C.

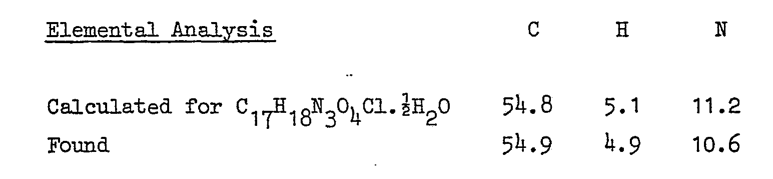

### Example 18 1-(4-chloxophenyl)-3-(1-ethoxycarbonyl)ethoxycarbonyl-6-methylpyridazin-4-one

1-(4-chlorophenyl)-3-carboxy-6-methylpyriaazin-4-one (2.6g) and carbonyldiimidazole (1.8g) were dissolved in methylene chloride and stirred for 1 hour at room temperature. Subsequently ethyl lactate (1.2g) was added and the mixture was stirred for 48 hours at 20⁰C afterwhich it was washed with water (200ml) and a sodium bicarbonate solution (200ml). The washed mixture was then dried over sodium sulphate and evaporated to dryness to afford, on recrystallization from ether, a white solid (1.3g) melting at 152-155°C.

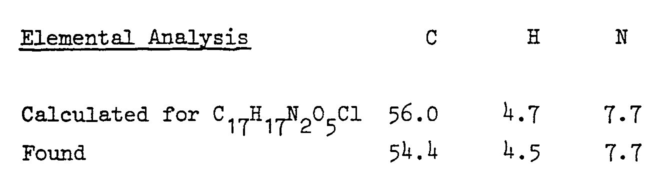

### Examples 19-21

The procedure of Example 18 was repeated using benzyl alcohol, phenol and 2-methylthioethanol, instead of ethyl lactate, in separate experiments except in the latter two cases the reactants were refluxed for 24 hours prior to water washing.

### Example 19 1-(4-chlorophenyl)-3-benzyloxycarbonyl-6-methyl- pyridazin-4-one

Melting point: 162-164°C.

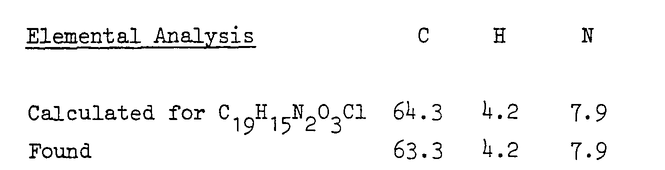

### Example 20 1-(4-chlorophenyl)-3-phenoxycarbonyl-6-methylpyrid- azin-4-one

Melting point: 146-148°C.

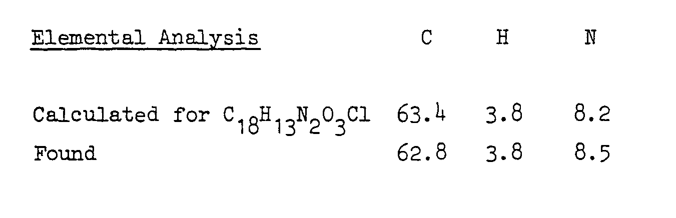

### Example 21 1-(4-dhlorophenyl)-3-(2-methylthio)ethoxycarbonyl-6-methylpyridazin-4-one

Melting point: 89-90°C.

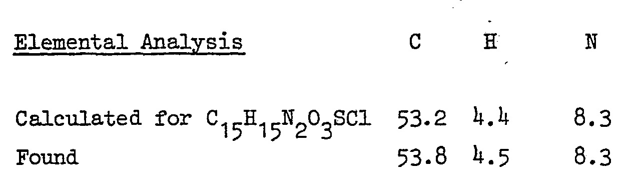

### Example 22 1-(4-chlorophenyl)-3-(2-butyloxy)ethoxycarbonyl-6-methylpyridazin-4-one

1-(4-chlorophenyl)-3-carboxy-6-methylpyridazin-4-one (5.3g) was dissolved in methylene chloride (120ml) and carbonyldiimidazole (4g) was added. This solution was stirred for 1 hour at room temperature and butane oxital (2.5g) in methylene chloride (25ml) was added. The reaction mixture was stirred for 24 hours at room temperature and washed with water and a saturated sodium bicarbonate solution, dried over sodium sulphate and evaporated down. Recrystallization from ethyl acetate (3 times) afforded 2.7g of a white solid melting at 80-82°C.

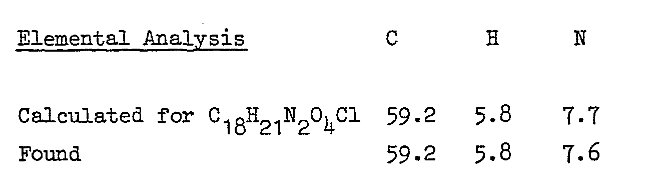

### Example 23 Demonstration of soyabean growth regulation

The plants used in the evaluation of the compounds were determinate soyabean cultivars (e.g. Fiskeby V).

Liquid formulations of the compounds were applied to plants at an early stage of flower development. The formulations used consisted of 60% water, 40% acetone which contained 0.4% TRITON X155 (Trade Mark), and amounts of the test compound to give a spray application eqivalent to 0.5, 1.0 and 2.0 Kg/ha.

In order to induce nodulation, the soyabean seeds were inoculated with a commercial strain of Rhizobium ("Nitrogerm", Root Nodule Pty., Ltd., Australia) prior to sowing in a loam: grit (5:1) mixture in 5" diameter pots. Plants were maintained at 2l-25⁰C under 14 hr. day length and watered by sub-irrigation. Treatments were as foliar applications to three plants for each dose of the test material, applied in a volume equivalent to 632 litres per hectare. After treatment the plants were set out in a randomised block design. After four weeks the total pod number per plant was recorded. Results were derandomised and mean values calculated and expressed as a percentage of the untreated controls. The pod numbers thus obtained are given in Table I.

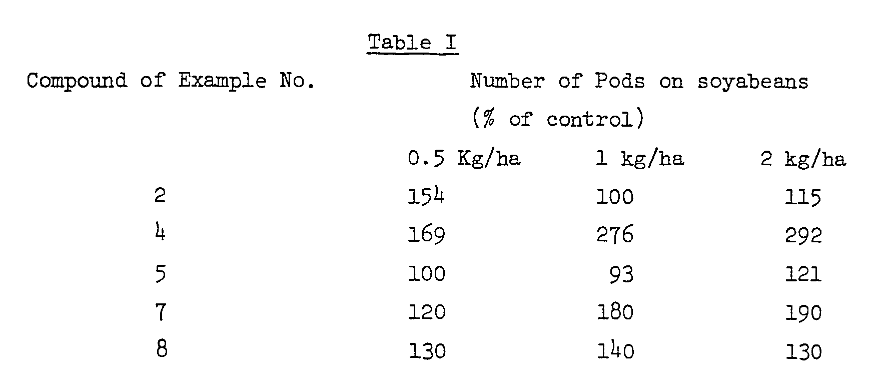

### Example 24 Demonstration of pollen-suppressing activity

Spring wheat, variety Sicco, was propagated in a glass-house in 8 cm pots containing a loam-based compost. Supplementary lighting was provided by high-pressure mercury vapour lamps to give a constant day length of 16 hours. The temperature was maintained at approximately 2₀⁰C.

The compounds to be tested were formulated as aqueous solutions containing 0.1% nonidet P 40 (trade mark) as wetting agent and 1% acetone to aid solubility. These formulations were diluted with water to a concentration of 1000 ppm and sprayed onto plants in a volume equivalent to 650 litres/ha. The plants were treated at the growth stage when the second node of the plant was just detectable.

At ear emergence but before anthesis, 3 heads from each main stem and 2 heads from the tillers in each pot were placed in cellophane bags to prevent cross-pollination. At maturity, the bagged ears were harvested, and seed set was recorded and compared with untreated controls.

The results are shown in Table II.

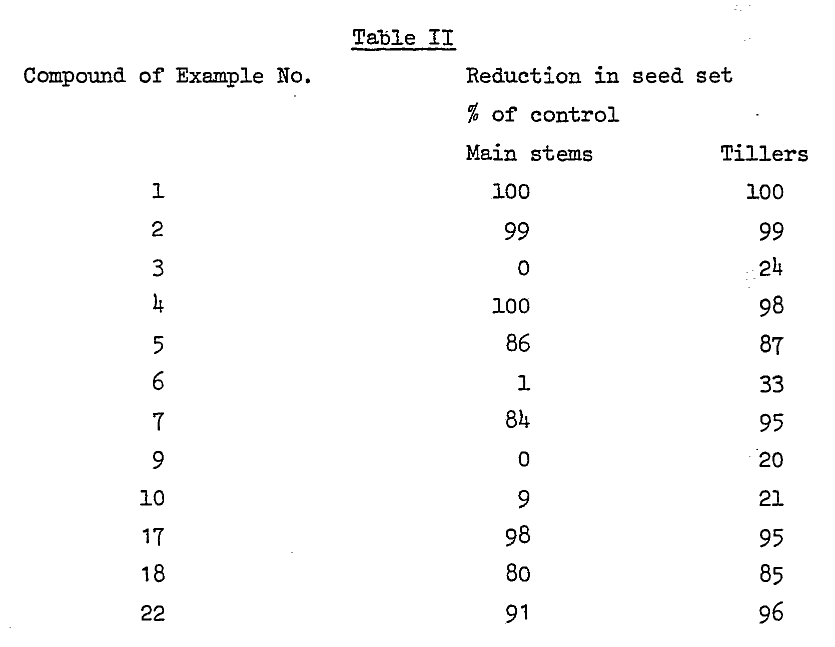

It can be seen that all the test compounds produced a considerable reduction in seed set compared with the untreated control, clearly illustrating the ability of the compounds to sterilize the male anthers of the wheat.

## Claims

1. A compound of the general formula 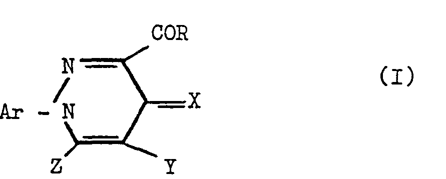 in which X represents an oxygen or sulphur atom;
Y represents a hydrogen or halogen atom or an alkyl group;
Z represents an alkyl group;
Ar represents an optionally-substituted phenyl group; and R represents a group
i) NR¹R² or ONR¹R², in which R¹ represents a hydrogen atom or an alkyl, alkenyl, alkynyl or cycloalkyl group and R² represents a hydrogen atom, an alkyl, alkenyl, alkynyl, cycloalkyl, alkoxy or hydroxy group, or an acyl group derived from a carboxylic or carbamic acid, or an alkyl group substituted with a carboxylic acid or ester group, or R¹ and R ² together with the interjacent nitrogen atom represent an optionally substituted heterocyclic ring; or
ii) OR in which R³ represents an alkenyl, alkynyl, cycloalkyl or aryl group or an alkyl, alkenyl, alkynyl, cycloalkyl or aryl group substituted by one or more of the same or different substituents selected from halogen atoms, alkoxy, alkylthio, cyano and aryl groups, acyl groups derived from carboxylic or carbamic acids, groups of formula NR¹R² where R and R have the meaning given above, and carboxy groups and salts and esters thereof; or
iii) _{SR}⁴ in which R⁴ has one of the meanings given above for R³ or represents a hydrogen atom or an alkyl group; or
iv) ON=_{CR}⁴_{R}⁵ in which R⁴ has the meaning given above and R⁵ has one of the meanings given above for R³ or represents an alkyl group, or R⁴ and R⁵ together with the interjacent carbon atom represent an optionally substituted carbocyclic or heterocyclic ring; or
ᵥ) _{NR}⁶_{NR}⁷_{R}⁸ in which each of R⁶ and R⁷ independently has one of the meanings given above for R¹ and R⁸ has one of the meanings given above for R or represents an acyl group derived from a carboxylic acid or R⁷ and _{R}⁸ together with the interjacent nitrogen atom represent an optionally substituted heterocyclic ring.

A compound as claimed in claim 1, in which X represents an oxygen atom.

A compound as claimed in either claim 1 or claim 2, in which Y represents a hydrogen atom and Z represents a methyl group.

A compound is claimed in any one of claims 1 to 3, in which Ar represents a phenyl group which is unsubstituted or substituted by one or more of the same or different substituents selected from halogen atoms and alkyl, alkoxy, alkylthio, cyano, aryl and carboxy groups.

A compound as claimed in claim 4, in which Ar represents a monohalophenyl group.

A compound as claimed in any one of claims 1 to 5, in which R represents a group
i) NR¹R² or ONR¹R², in which R represents a hydrogen atom or an alkyl or cycloalkyl group, and R ² represents a hydrogen atom, an alkyl, alkoxy or hydroxy group, or a group of formula COR⁹ or CO.NHR⁹ in which R⁹ represents an alkyl or cycloalkyl group, or -CH-COOR¹¹ in which R represents a hydrogen atom or an alkyl group and R¹¹ is a hydrogen atom or an alkyl group, or R¹ and _{R}² together with the interjacent nitrogen atom represent a 5 or 6 membered heterocyclic ring having 1, 2 or 3 nitrogen atoms; or
ii) OR³ in which R³ represents an alkenyl group having up to 4 carbon atoms, a cycloalkyl group having 3, 5 or 6 carbon atoms, or an alkyl or alkenyl group substituted by one or more halogen atoms, by a group of formula C_{OR}⁹ where R⁹ has the meaning given in (i) in this claim, by a phenyl group, by an alkoxy group, by an NR¹R² group where R and R² have the meanings given in (i) either in claim 1 or in this claim, or by a carboxy group or a salt or ester thereof; or
iii) SR⁴ in which R⁴ has one of the meanings given for R³ in (ii) of this claim, or represents a hydrogen atom or an alkyl group; or
iv) ON=_{CR}⁴_{R}⁵ in which R⁴ has the meaning given in (iii) in this claim and R has one of the meanings given for R³ in (ii) in this claim or represents an alkyl group; or
ᵥ) _{NR}⁶_{NR}⁷_{R}⁸ in which each of R⁶, R7 and R⁸ independently has one of the meanings given for R¹ in (i) in this claim, or R⁶ has said meaning and R⁷ and R⁸ together with the interjacent nitrogen atom represent a heterocyclic ring having 1, 2 or 3 nitrogen atoms.

A compound as claimed in claim 6, in which R represents a group
i) NR¹R² or ONR¹R² in which R¹ represents a hydrogen atom or a methyl or cyclohexyl group, and R represents a hydrogen atom, a methyl, methoxy or hydroxy group, or a group of formula COR⁹ or CONHR⁹ in which R⁹ represents a methyl or cyclohexyl group, or R ¹ or R² together represent an imidazole or triazole ring; or
ii) OR³ in which R³ represents an allyl group, a benzyl group, a cyclohexyl group, a monohaloalkyl group, or a group -CH(CH₃)- or -(CH₂)ₙ- where n is an integer 1 to 4, bearing an alkylcarbonyl group, an alkoxy group, an NR¹R² group where R¹ and R² have the meanings given in (i) in either claim 1 or claim 6 or this claim, or a carboxy group or a salt or an ester thereof; or
iii) SR⁴ in which R4 has one of the meanings given for R³ in (ii) of this claim or represents a hydrogen atom or a methyl, ethyl or isopropyl group; or
iv) ON=CR⁴R⁵ in which R⁴ has the meaning given in (iii) in this claim and R has one of the meanings given for R³ in (ii) in this claim or represents a methyl group; or
ᵥ) NR⁶NR⁷R⁸ in which each of R⁶, R⁷ and R8 independently has one of the meanings given for R¹ in (i) in this claim, or R has said meaning and R and R together with the interjacent nitrogen atom represent an imidazole or triazole ring.

A compound as claimed in claim 7, in which R represents a group of general formula
i) NR¹R² in which R¹ represents a hydrogen atom or a methyl or cyclohexyl group, and R² represents a methoxy group or a -CO.NHcyclohexyl group; or R¹ and R² together with the interjacent nitrogen atom represents an imidazole ring; or
ii) OR³ in which R³ represents an allyl, 2-chloroethyl, 2-bromoethyl or methylcarbonylmethyl group; or
iii) SR⁴ in which R4 represents an isopropyl or cyclohexyl group; or
iv) ON=_{CR}⁴_{R}⁵ in which each of R⁴ and R⁵ represents a methyl group; or
ᵥ) NR⁶NR⁷R⁸ in which _{R}⁶ represents a hydrogen atom and each of R⁷ and R⁸ represents a hydrogen atom or a methyl group.

A process for the preparation of a compound as claimed in claim 1, in one or more steps, which involves converting a compound of the general formula 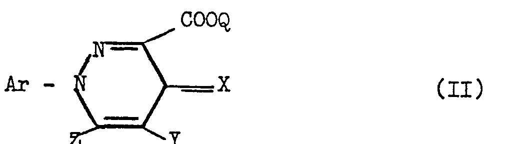 in which Ar, X, Y and Z have the meanings given in claim 1 and Q represents a hydrogen atom, a cation or an alkyl group, into a compound as claimed in claim 1, by reaction with a compound capable of replacing the OQ group with a group R where R has the meaning given in claim 1.

A process as claimed in claim 9, which comprises a first step in which the compound of formula II is converted into the corresponding acid halide, a mixed anhydride with an ester of formic acid, or a compound of formula I in which R represents an N-bonded imidazole group.

A process as claimed in either claim 9 or claim 10, in which the compound capable of replacing the OQ group with a group R is:
i) a compound of formula NHR¹R² or HONRK ; or
ii) a compound of formual R³0H or R³Hal where Hal is a halogen atom; or
iii) a compound of formula R⁴SH;
iv) a compound of formula HON=CR⁵R⁶ or an alkali metal salt thereof; or
v) a compound of formula HNR⁷NR⁸R⁹.

A compound as claimed in claim 1, whenever prepared by a process as claimed in any one of claims 9 to 11.

A plant growth regulating composition which comprises a compound as claimed in any one of claims 1 to 8 and 12 together with a carrier.

A composition as claimed in claim 13, which comprises at least two carriers, at least one of which is a surface-active agent.

A method of regulating the growth of a plant, which comprises treating the plant, the seed from which it is to be grown, or the medium in which it is growing or is to be grown, with a compound as claimed in any one of claims 1 to 8 and 12 or a composition as claimed in either claim 13 or claim 14.

A method as claimed in claim 15, which comprises increasing the yield of soya beans by treating a soya bean plant or the soil in which it is growing with a compound as claimed in any one of claims 1 to 8 and 12 or a composition as claimed in either claim 13 or claim 14, at a growth stage after the flowers of the plant have differentiated.

A method as claimed in claim 15, which comprises sterilizing the male anthers of a plant by treating the plant or the soil in which it is growing with a compound as claimed in any one of claims 1 to 8 and 12 or a composition as claimed in either claim 13 or claim 14, at a growth stage such that sterilization occurs.

A method as claimed in claim 17, in which the plant is a small-grain cereal plant.

A method of producing F₁ hybrid seed which comprises cross-pollinating a plant which has been treated by a process as claimed in either claim 17 or claim 18, with a second plant of a different strain.
